(19) Europäisches Patentamt
European Patent Office
Office européen des brevets

(11) Publication number: 0 246 735
A1

(12) EUROPEAN PATENT APPLICATION

(21) Application number: 87302346.9

(22) Date of filing: 18.03.87

(51) Int. Cl.³: C 07 D 275/02
C 07 D 417/06, A 01 N 43/80
C 07 F 9/65

(30) Priority: 18.04.86 GB 8609538

(43) Date of publication of application:
25.11.87 Bulletin 87/48

(84) Designated Contracting States:
AT BE DE ES FR GB GR IT LU

(71) Applicant: IMPERIAL CHEMICAL INDUSTRIES PLC
Imperial Chemical House Millbank
London SW1P 3JF(GB)

(72) Inventor: Godfrey, Christopher Richard Ayles
159 Viking Great Hollands
Bracknell Berkshire(GB)

(74) Representative: Alner, Henry Giveen Hamilton et al,
Imperial Chemical Industries PLC Legal Department:
Patents P.O. Box 6 Bessemer Road
Welwyn Garden City Herts, AL7 1HD(GB)

(54) Heterocyclic compounds.

(57) Compounds of formula:

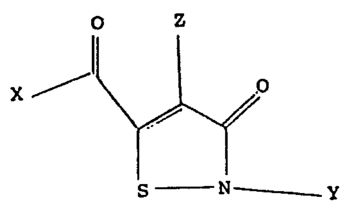

wherein X is an optionally substituted aryl (especially phenyl), aralkyl, alkyl, hydroxy, alkoxy, aryloxy, alkylamino, dialkylamino, or heterocyclyl group; or a hydrogen atom; Z is hydrogen, alkyl, cyano or halogen; and Y is an optionally substituted aralkyl, alkyl, substituted alkyl, or aryl group or a hydrogen atom; provided that when X is phenyl and Z is hydrogen Y is not methyl, phenyl 4-methylphenyl, 4-hydroxyphenyl, 4-methoxyphenyl, 4-chlorophenyl, 3- or 4-nitro phenyl, or benzyl; have fungicidal activity.

EP 0 246 735 A1

# HETEROCYCLIC COMPOUNDS

This invention relates to heterocyclic compounds useful as fungicides, to processes for preparing them, to fungicidal compositions containing them, and to a method of combating fungi, especially fungal infections in plants.

The invention provides a compound having the general formula (I):

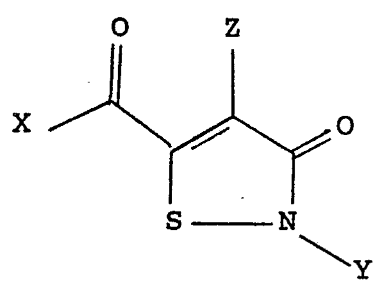

wherein X is an optionally substituted aryl (especially phenyl), aralkyl, alkyl, hydroxy, alkoxy, aryloxy, amino, alkylamino, dialkylamino, or a heterocyclic group; or a hydrogen atom; Y is an optionally substituted aralkyl (especially benzyl), aryl, alkenyl, alkyl or substituted alkyl group, or a hydrogen atom; and Z is an alkyl or cyano group, or a hydrogen or halogen atom; provided that when X is phenyl and Z is hydrogen Y is not methyl, phenyl, 4-methylphenyl, 4-hydroxyphenyl, 4methoxyphenyl, 4-chlorophenyl, 3- or 4-nitrophenyl, or benzyl.

Examples of suitable substituent groups for X and Y when they represent aryl or aralkyl groups, especially phenyl or benzyl respectively, are halogen, alkyl, haloalkyl, alkoxy, cyano and nitro. The aryl or aralkyl groups, which are preferably phenyl or benzyl, may be unsubstituted or substituted with 1,2 or 3 substituents,

which may be the same or different, as defined above. Examples of X and Y are phenyl; 2-,3- or 4-chlorophenyl; 2,3-, 2,6-, 3,4- or 3,5- dichlorophenyl; 2-,3- or 4-fluorophenyl; 2,4-, 2,6-, 3,4- or 3,5- difluorophenyl; 2-,3- or 4- bromophenyl; 2,3- or 4- methoxyphenyl; 2,4-, 2,6-, 3,4-, or 3,5- dimethoxyphenyl; 2-,3- or 4-nitrophenyl; 2-,3- or 4- methylphenyl; 2-,3- or 4-trifluoromethylphenyl; benzyl; 2-,3- or 4- chlorobenzyl; 2,4-, 2,6-, 3,4- or 3,5-dichlorobenzyl; 2-, 3- or 4-fluorobenzyl; 2,4-, 2,6-, 3,4-, 3,5- difluorobenzyl; 2-, 3-, or 4-bromobenzyl; 2-, 3- or 4- methoxybenzyl; 2,4-, 2,6-, 3,4-, or 3,5- dimethoxybenzyl; 2-, 3- or 4-nitrobenzyl; 2-, 3- or 4- methylbenzyl; and 2-, 3- or 4-trifluoromethylbenzyl; 2- or 3- pyridylmethyl; 2- or 3-furanylmethyl; and 2- or 3- thiophenylmethyl.

When X, Y or Z is alkyl or contains an alkyl moiety as in alkoxy alkylamino or dialkylamino, the alkyl moiety can be a cyclic, straight or branched chain alkyl group having 1 to 10 carbon atoms or any combination thereof. Thus the term "alkyl" in this specification is intended to include alkyl, cycloalkyl and cycloalkylalkyl groups. Examples are methyl, ethyl, propyl (n-or iso-propyl) and butyl (n-, sec-, iso- or t-butyl). Suitable substituents for X, Y and Z when these are alkyl groups include halogen, aryl, alkoxy and hydroxy.

Examples of the compounds of the invention which conform to formula (I) are shown in Table I.

TABLE I

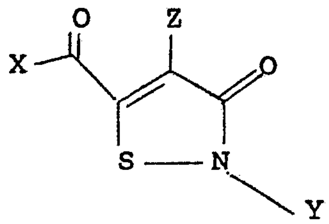

| COMPOUND NUMBER | X | Y | Z | M.pt. (°C) |
|---|---|---|---|---|
| 1 | $C_6H_5$- | $C_6H_5CH(CH_3)$- (R,S) | H | Oil |
| 2 | $C_6H_5$- | 2-$CH_3O$-$C_6H_4CH_2$- | H | 126-128 |
| 3 | $C_6H_5$- | 2-Cl-$C_6H_4CH_2$- | H | 128-131 |
| 4 | $C_6H_5$- | 2-F-$C_6H_4CH_2$- | H | 112-115 |
| 5 | $C_6H_5$- | 4-$CH_3$-$C_6H_4CH_2$- | H | 128-130 |
| 6 | $C_6H_5$- | 4-$CH_3O$-$C_6H_4CH_2$- | H | 101-104 |
| 7 | $C_6H_5$- | 4-Cl-$C_6H_4CH_2$- | H | 126-128 |
| 8 | $C_6H_5$- | 3-F-$C_6H_4CH_2$- | H | 112-115 |
| 9 | $C_6H_5$- | 3-$CH_3O$-$C_6H_4CH_2$- | H | 85-89 |
| 10 | $C_6H_5$- | 3,4-di-Cl-$C_6H_3CH_2$- | H | 132-134 |
| 11 | $C_6H_5$- | 2,4,-di-Cl-$C_6H_3CH_2$- | H | 133-136 |
| 12 | $C_6H_5$- | 3,4-di-$CH_3O$-$C_6H_3CH_2$- | H | 138-140 |
| 13 | $C_6H_5$- | 3-$CF_3$-$C_6H_4CH_2$- | H | 98-99 |
| 14 | $C_6H_5$- | 4-F-$C_6H_4CH_2$- | H | 132-133 |
| 15 | $C^6H_5$- | $C_6H_5CH(CH_3)$- (S) | H | 89-90 |
| 16 | $C_6H_5$- | $C_6H_5CH(CH_3)$- (R) | H | 83-86 |
| 17 | $C_6H_5$- | $C_6H_5CH_2$- | Cl | 90-91 |
| 18 | $C_6H_5$- | 4-Br-$C_6H_4CH_2$- | H | 123-126 |
| 19 | $C_6H_5$- | $C_6H_5CH_2$- | $CH_3$ | Oil |
| 20 | $C_6H_5$- | 3,4-Methylenedioxy-$C_6H_3CH_2$- | H | 121-123 |

TABLE I (CONT/D)

| COMPOUND NUMBER | X | Y | Z | M.pt. (°C) |
|---|---|---|---|---|
| 21 | $C_6H_5$- | 4-$NO_2$-$C_6H_4CH_2$- | H | 161-162 |
| 22 | $C_6H_5$- | 2,6-di-$CH_3$-$C_6H_3$- | H | 147-148 |
| 23 | $C_6H_5$- | $C_6H_5CH_2$- | Br | Oil |
| 24 | $C_6H_5$- | 2,6-di-$CH_3$-$C_6H_3$- | Cl | Oil |
| 25 | $C_6H_5$- | 4-$NO_2$-$C_6H_4$-$CH_2$- | Cl | Oil |
| 26 | $C_6H_5$- | 3,5-di-Cl-$C_6H_3$- | H | 225-227 |
| 27 | $C_6H_5$- | t-$C_4H_9$ | H | Oil |
| 28 | $C_6H_5$- | $CH_2$=C($CH_3$)$CH_2$- | H | 67-70 |
| 29 | $C_6H_5$- | n-$C_4H_9$- | H | Oil |
| 30 | $C_6H_5$- | $C_2H_5$- | H | 76-78 |
| 31 | $C_6H_5$- | $C_2H_5CO_2CH_2$- | H | 108-109 |
| 32 | $C_6H_5$- | $CH_2$=$CHCH_2$- | H | 61-66 |
| 33 | $C_6H_5$- | $C_2H_5OCOCH(CH_3)$- | H | 76-80 |
| 34 | $C_6H_5$- | n-$C_6H_{13}$ | H | Oil |
| 35 | $C_6H_5$- | cyclo-$C_6H_{11}$- | H | 90-94 |
| 36 | $C_6H_5$- | iso-$C_3H_7$- | H | 78-79 |
| 37 | $C_6H_5$- | cyclo-$C_6H_{11}CH_2$- | H | 78-79 |
| 38 | $C_6H_5$- | $C_6H_5$-$CH_2CH_2$- | H | 117-118.5 |
| 39 | $C_6H_5$- | $CH_3CO.OCH_2CH_2$- | H | Oil |
| 40 | $C^6H_5$- | $HOCH_2CH_2$- | H | 105-106 |
| 41 | $C_6H_5$- | 1-Adamantyl | H | 154-156 |
| 42 | $C_6H_5$- | $C_6H_5CH_2CH(CO_2CH_3)$- | H | Oil |
| 43 | $C_6H_5$- | 2-Furanylmethyl | H | 103-106 |
| 44 | $C_6H_5$- | 2-thiophenylmethyl | H | 95-97 |
| 45 | $C_6H_5$- | 2-pyridylmethyl | H | 93-95 |

TABLE I (CONT/D)

| COMPOUND NUMBER | X | Y | Z | M.pt. (°C) |
|---|---|---|---|---|
| 46 | $C_6H_5$- | 3-pyridylmethyl | H | 137-139 |
| 47 | 4-Cl-$C_6H_4$- | $C_6H_5CH_2$- | H | 150-151 |
| 48 | 4-F-$C_6H_4$- | $C_6H_5CH_2$- | H | 137-138 |
| 49 | 4-$CH_3O$-$C_6H_4$- | $C_6H_5CH_2$- | H | 140-141 |
| 50 | 4-$CH_3O$-$C_6H_4$- | $C_6H_5CH_2$- | Cl | Oil |
| 51 | 3-$CH_3$-$C_6H_4$- | $C_6H_5CH_2$- | H | 90-93 |
| 52 | 2-$CH_3$-$C_6H_4$- | $C_6H_5CH_2$- | H | 50-53 |
| 53 | 3-F-$C_6H_4$- | $C_6H_5CH_2$- | H | 132-134 |
| 54 | 3-Cl-$C_6H_4$- | $C_6H_5CH_2$- | H | 115-117 |
| 55 | 2,4-di-Cl-$C_6H_3$- | $C_6H_5CH_2$- | H | 99-103 |
| 56 | 2,4-di-F-$C_6H_3$- | $C_6H_5CH_2$- | H | 96-98 |
| 57 | 4-$CH_3$-$C_6H_4$- | $C_6H_5CH_2$- | H | 138-139 |
| 58 | $CH_3O$- | $C_6H_5CH_2$- | H | 102-105 |
| 59 | HO- | $C_6H_5CH_2$- | H | 199-200 |
| 60 | $CH_3O$- | 4-Cl-$C_6H_4CH_2$- | H | 108-111 |
| 61 | $C_2H_5O$- | $C_6H_5CH_2$- | Cl | Oil |
| 62 | $C_2H_5O$- | $C_6H_5CH_2$- | H | 80-83 |
| 63 | $C_6H_5CH_2O$- | 4-Cl-$C_6H_4$-$CH_2$- | H | 100-102 |
| 64 | 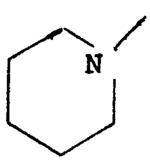 | 4-Cl-$C_6H_4$-$CH_2$- | H | 97-99 |
| 65 | $CH_3O$- | $C_6H_5CH_2$- | Cl | Oil |
| 66 | 2-Furanyl | $C_6H_5CH_2$- | H | 140-144 |

TABLE I (CONT/D)

| COMPOUND NUMBER | X | Y | Z | M.pt. (°C) |
|---|---|---|---|---|
| 67 | 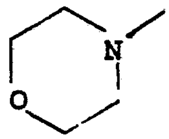 | 4-Cl-$C_6H_4CH_2$- | H | 108-111 |
| 68 | iso-$C_3H_7$-NH- | 4-Cl-$C_6H_4$-$CH_2$- | H | 187-190 |
| 69 | $C_6H_5$ | $(C_2H_5O)_2\overset{O}{\overset{\|}{P}}CH_2$- | H | Oil |
| 70 | 4-Cl-$C_6H_4$- | $C_6H_5CH_2$- | H | 101-104 |

The compounds of the invention having the general formula (I):

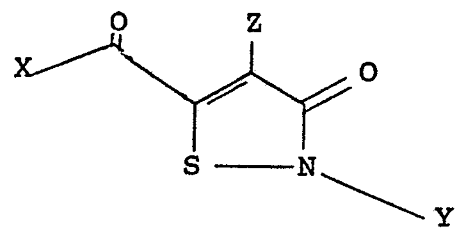

(I)

wherein X is an optionally substituted aryl (especially phenyl), aralkyl, alkyl, cycloalkyl or heterocyclic group as defined above, and Y and Z are as defined above, can be prepared by treatment of gamma-keto-amides of general formula (II):

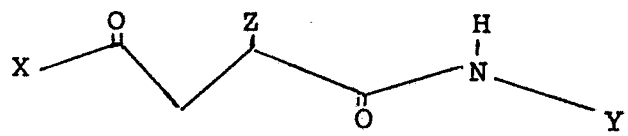

(II)

wherein X is an optionally substituted aryl (especially phenyl), aralkyl, alkyl, cycloalkyl or heterocyclic group as defined above, and Y and Z are as defined above, with thionyl chloride in the presence or absence of an acid-binding agent (such as potassium carbonate or sodium bicarbonate) with or without a convenient co-solvent (such as chloroform or dichloromethane) and at a convenient temperature (such as 0 to 80°C) (see, for example, A. Tsolomitis, C. Sandris, J. Heterocyclic Chem, 1980, 17, 1645, RJS. Beer, D. Wright, Tetrahedron, 1981, 37, 3867).

Gamma-keto-amides of the general formula (II) can be prepared from gamma-keto-acids of general formula (III):

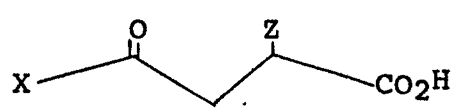

(III)

wherein X and Z are as defined above, by treatment with amines of general formula (IV):

$Y\text{-}NH_2$ (IV)

wherein Y is as defined above, using standard methods as set out in the chemical literature.

Gamma-keto-acids of the general formula (III) can be prepared from cyclic anhydrides of general formula (V):

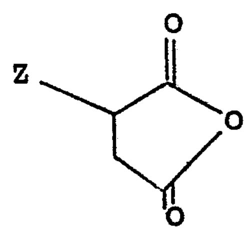

(V)

wherein Z is as defined above (excluding halogen) by treatment with Grignard reagents of general formula (VI):

X-Mg-A (VI)

wherein X is as defined above and A is halogen, or with organolithium reagents of general formula (VII):

X-Li (VII)

wherein X is as defined above, in a suitable solvent (such as diethyl ether or tetrahydrofuran) at low temperatures (such as -78°C).

Alternatively, gamma-keto-acids of general formula (III) can be prepared from gamma-keto-esters of general formula (VIII):

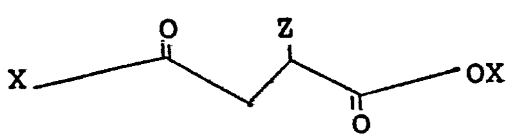

(VIII)

wherein X is as defined above and Z is as defined above (but is not halogen) by hydrolysis using standard methods as set out in the chemical literature.

Gamma-keto-esters of general formula (VIII) can be prepared from aldehydes of general formula (IX):

X-CHO (IX)

wherein X is as defined above, by treatment with an ester of general formula (X):

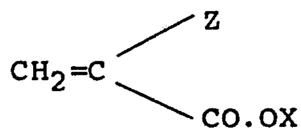

(X)

wherein X and Z are as defined above, in a suitable solvent (such as dimethylformamide) in the presence of a suitable catalyst (such as potassium cyanide) (see, for example, H. Stetter, Angew Chemie (Int Ed), 1976, 15, 639).

Alternatively, gamma-keto-acids of general formula (III) can be prepared from compounds of general formula (XI):

X-H (XI)

wherein X is an optionally substituted aryl (especially phenyl) as defined above or alkenyl group, by treatment with an anhydride of general formula (V) in a convenient solvent (such as nitrobenzene or nitromethane) in the presence of a suitable catalyst (such as aluminium chloride).

In addition, gamma-keto-amides of general formula (II) may be prepared from cyclic imides of general formula (XII):

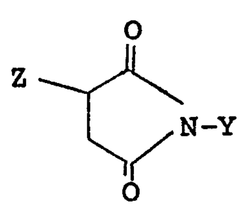

(XII)

wherein Y is as defined above and Z is as defined above (but is not halogen) by treatment with organolithium reagents of general formula (VII) or Grignard reagents of general formula (VI) in a suitable solvent (such as diethyl ether or tetrahydrofuran) at low temperatures (such as -78°C).

Cyclic imides of general formula (XII) may be prepared from dicarboxylic acids of general formula (XIII):

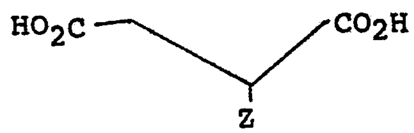

(XIII)

wherein Z is as defined above (but is not halogen) and amines of general formula (IV) using standard methods as set out in the chemical literature.

In an alternative approach, compounds of general formula (I) may be prepared from compounds of general formula (I) wherein X and Z are as defined above and Y is a hydrogen atom, or in the form of one of its alkali metal salts, by treatment with a compound of general formula (XIV):

Y-A (XIV)

wherein A and Y are as defined above, in a suitable solvent (such as dimethyl formamide).

The compounds of the invention having the general formula (I), wherein X is an alkoxy, aryloxy, alkylamino or dialkylamino group, and Y and Z are as defined above, can be prepared from carboxylic acids of the general formula (XV):

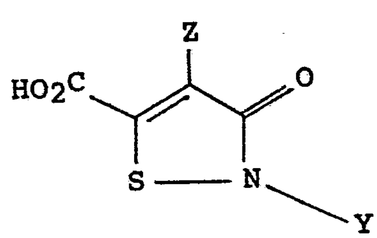

(XV)

wherein Y and Z are as defined above, by standard methods as set out in the chemical literature.

Carboxylic acids of general formula (XV) can be prepared from esters of general formula (XVI):

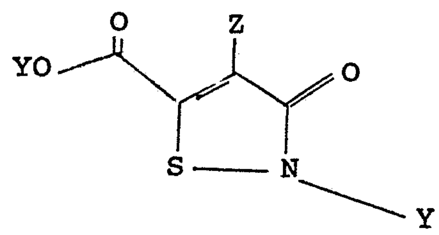

(XVI)

wherein Y and Z are as defined above, by standard methods as set out in the chemical literature.

Esters of general formula (XVI) can be prepared from compounds of general formula (XVII):

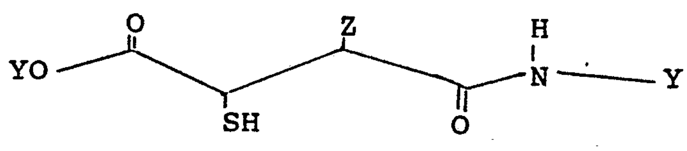

(XVII)

wherein Y is as defined above and Z is as defined above (but is not halogen), by treatment with sulphuryl chloride in the presence or absence of an acidbinding agent (such as potassium carbonate or sodium bicarbonate) in a suitable solvent (such as chloroform or dichloromethane) at a suitable temperature (such as 0° to 60°C).

Compounds of general formula (XVII) can be prepared from compounds of general formula (XVIII):

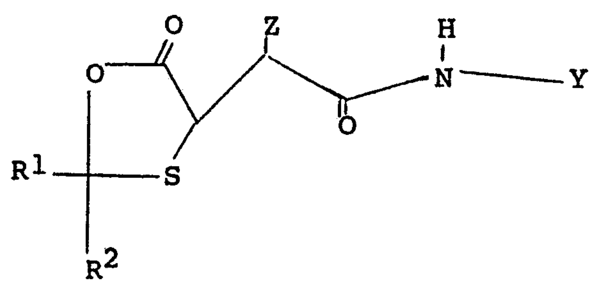

(XVIII)

wherein $R^1$ and $R^2$ are alkyl or aryl groups, and Y is as defined above and Z is as defined above (but is not halogen) by treatment with an alcohol of general formula (XIX):

Y-OH (XIX)

wherein Y is as defined above, in the presence of an acid catalyst (such as hydrogen chloride) with or without a convenient co-solvent.

Compounds of general formula (XVIII) may be prepared from carboxylic acids of general formula (XX):

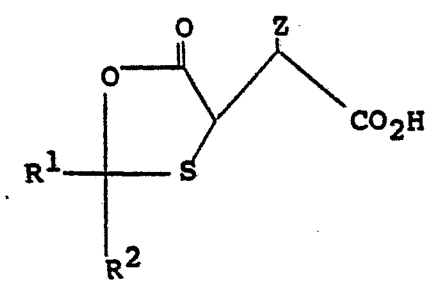

(XX)

wherein $R^1$ and $R^2$ are as defined above and Z is as defined above (but is not halogen) by treatment with amines of general formula (IV) using standard methods as set out in the chemical literature.

Compounds of general formula (XX) can be prepared from dicarboxylic acids of general formula (XXI):

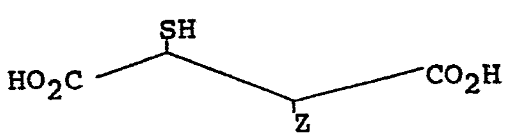

(XXI)

wherein Z is as defined above (but is not halogen) by treatment with compounds of general formula (XXII):

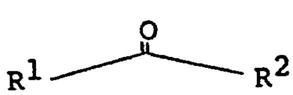

(XXII)

wherein $R^1$ and $R^2$ are as defined above, in a suitable solvent (such as toluene), in the presence of an acid catalyst (such as p-toluenesulphonic acid) at a suitable temperature (such as 50 to 120°C).

Alternatively, compounds of general formula (XVI) may be prepared from compounds of general formula (XXIII):

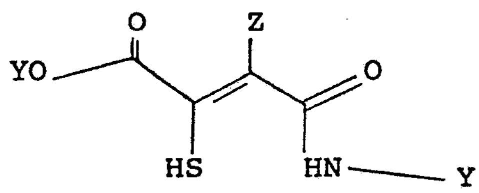

(XXIII)

wherein Y and Z are as defined above, by treatment with sulphuryl chloride in a convenient solvent (chloroform or dichloromethane) with or without an acid-binding agent.

Compounds of general formula (XXIII) may be prepared from compounds of general formula (XXIV):

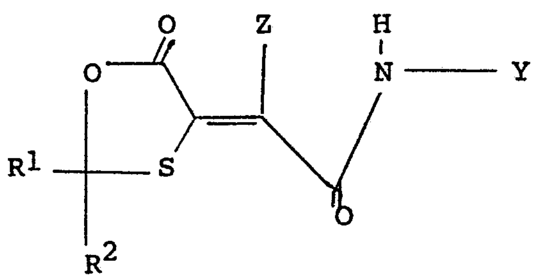

(XXIV)

wherein $R^1$, $R^2$, Y and Z are as defined above, by treatment with alcohols of the general formula (XIX) or in the form of one of the alkali metal salts, in a suitable solvent.

Compounds of general formula (XXIV) may be prepared from carboxylic acids of general formula (XXV):

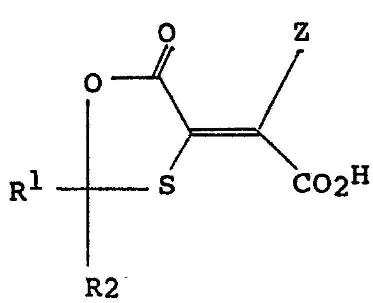

(XXV)

wherein $R^1$, $R^2$ and Z are as defined above, by treatment with amines of general formula (IV) using standard methods as set out in the chemical literature.

Carboxylic acids of general formula (XXV) can be prepared from carboxylic acids of general formula (XX), by treatment with sulphuryl chloride in a convenient solvent (such as dichloromethane or chloroform) at a convenient temperature (such as 20 to 69°C).

The compounds of the invention having the general formula (I), wherein X and Y are as defined above, and Z is a halogen atom, can be prepared from compounds of general formula (XXVI):

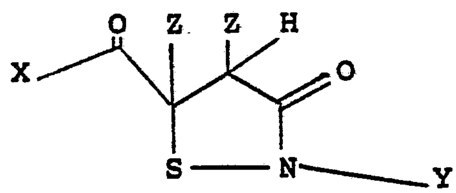

(XXVI)

wherein X and Y are as defined above, and Z is a halogen atom, by treatment with an acid-binding agent (such at triethlamine) in a convenient solvent (such as chloroform or dichloromethane).

Compounds of general formula (XXVI) can be prepared from compounds of general formula (I) wherein X and Y are as defined above and Z is a hydrogen atom, by treatment with a halogenating agent in a convenient solvent (such as chloroform or dichloromethane).

The compounds, salts and metal complexes are active fungicides, particularly against the diseases:-

*Plasmopara viticola* on vines
*Piricularia oryzae* on rice
*Puccinia recondita*, *Puccinia striiformis* and other rusts on wheat, *Puccinia hordei*, *Puccinia striiformis* and other rusts on barley, and rusts on other hosts eg. coffee,

Cercospora arachidicola on peanuts and other Cercospora species on for example sugar beet, bananas and soya beans
Botrytis cinerea (grey mould) on tomatoes, strawberries, vines and other hosts
Venturia inaequalis (scab) on apples

Some of the compounds have also shown a broad range of activities against fungi in vitro.

The compounds can move acropetally in the plant tissue. Moreover, the compounds can be volatile enough to be active in the vapour phase against fungi on the plant.

They may also be useful as industrial (as opposed to agricultural) fungicides, eg. in the prevention of fungal attack of wood, hides, leather and especially paint films.

The compounds may be used as such for fungicidal or plant growth regulating purposes but are more conveniently formulated into compositions for such usage. The invention thus provides a fungicidal or plant growth regulating composition comprising a compound of general formula (I) as hereinbefore defined, or a salt, metal complex, ether or ester thereof; and, optionally, a carrier or diluent.

The invention also provides a method of combating fungi, which comprises applying to a plant, to seed of a plant, or to the locus of the plant or seed, a compound, or salt, metal complex, ether or ester thereof, as hereinbefore defined.

The compounds, salts, metal complexes, ethers and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems,

branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a

The compounds, salts, metal complexes, ethers and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the

plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form

aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg. of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, eg. compounds having similar or complementary fungicidal or plant growth activity or compounds having plant growth regulating, herbicidal or insecticidal activity.

The other fungicidal compound can be, for example, one which is capable of combating ear diseases of cereals (eg. wheat) such as Septoria, Gibberella and Helminthosporium spp., seed and soil borne diseases and downy and powdery mildews on grapes and powdery mildew and scab on apples etc. These mixtures of fungicides can have a broader spectrum of activity than the compound of general formula (I) alone; further the other fungicide can have a synergistic effect on the fungicidal activity of the compound of general formula (I). Examples of the other fungicidal compounds are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl,

fosetyl-aluminium, carboxin, oxycarboxin, fenarimol, nuarimol, fenfuram, methfuroxam, nitrothal-isopropyl, triadimefon, thiabendazole, etridiazole, triadimenol, biloxazol, dithianon, binapacryl, quinomethionate, guazatine, dodine, fentin acetate, fentin hydroxide, dinocap, folpet, dichlofluanid, ditalimphos, kitazin, cycloheximide, diclobutrazol, a dithiocarbamate, a copper compound, a mercury compound, 1-(2-cyano-2-methoxyiminoacetyl)-3-ethyl urea, fenapanil, ofurace, pro-piconazole, etaconazole and fenpropemorph.

The compounds of general formula (I) can be mixed with soil, peat or other rooting media for the protection of plants against seed-borne, soil-borne or foliar fungal diseases.

Suitable insecticides are Pirimor, Croneton, dimethoate, Metasystox and formothion.

The following Examples illustrate the invention; the temperatures are given in degrees Centrigrade (°C).

## EXAMPLE 1

This Example illustrates the preparation of 5-benzoyl-2-(4'-chlorobenzyl)-3-isothiazolinone (Compound No 7 of Table I).

To a solution of benzoylpropionic acid (5g) and triethylamine (4ml) in dry chloroform (50ml) at 0°C was added ethyl chloroformate (2.7ml) dropwise <u>via</u> syringe. After stirring for 10 minutes, a solution of 4-chlorobenzylamine (3.5ml) in chloroform (25ml) was added. The reaction was exothermic and a thick precipitate formed. After a further 10 minutes, the reaction mixture was diluted with chloroform and then washed successively with dilute hydrochloric acid and aqueous sodium bicarbonate. The organic phase was separated, dried and concentrated to give a solid (8.1g). Recrystallisation from aqueous methanol gave N-(4-chlorobenzyl)-benzoylpropionanide (7.4g, 87%), Mp 149-151°C.

A solution of N-(4-chlorobenzyl)-benzoylpropionamide (5g) in thionyl chloride (50ml) was stirred at room temperature. After 6 hours, the excess thionyl chloride was evaporated to give a dark brown oil. Toluene was added and then evaporated to remove the last traces of thionyl chloride. The dark residue was dissolved in ethanol (100ml) at reflux, and the resulting solution left to stand at -15°C overnight. The yellow crystals which formed were filtered and recrystallised from hot ethanol (75ml) to give 5-benzoyl-2-(4'-chlorobenzyl)-3-isothiazolinone (3.64g, 67%), Mp 126-128°C.

EXAMPLE 2

This Example illustrates the preparation of 2-benzyl-4-chloro-5-(4'-chlorobenzoyl)-3-isothiazolinone (Compound No 70 of Table I).

To a solution of 3-(4'-chlorobenzoyl)-propionic acid (5.97g) and triethylamine (4ml) in dry chloroform (50ml) at 0°C was added ethyl chloroformate (2.7ml) dropwise via syringe. After stirring for 10 minutes, a solution of benzylamine (3g) in dry chloroform (25ml) was added. After a further 10 minutes, the reaction mixture was diluted with chloroform and then washed successively with dilute hydrochloric acid and aqueous sodium bicarbonate. The organic phase was separated, dried and evaporated to give N-benzyl-3-(4'-chlorobenzoyl)-propionamide (6.5g). A suspension of N-benzyl-3-(4'-chlorobenzoyl)-propionamide (6g) in thionyl chloride (60ml) was stirred at room temperature for 24 hours. The resultant green solution was evaporated to dryness and the residue crystallised twice from hot ethanol to give yellow crystals of 2-benzyl-5-(4'-chlorobenzoyl)-3-isothiazolinone (3.4g), Mp 150-151°C.

A solution of 2-benzyl-5-(4'-chlorobenzoyl)-3-isothiazolinone (500mg) in sulphuryl chloride (7ml) was stirred at room temperature for 3 hours. The excess

sulphuryl chloride was removed in vacuo and the residue dissolved in toluene and re-evaporated. The resulting oil was re-dissolved in dichloromethane (10ml) and treated with 1,8-diazabicyclo [5.4.0] undec-7-ene (DBU) (0.25ml). After the reaction was complete (TLC analysis) the reaction mixture was washed with dilute hydrochloric acid. The organic phase was dried and evaporated and the residue separated by column chromatography to give 2-benzyl-4-chloro-5-(4'-chlorobenzoyl)-3-isothiazolinone as a yellow oil (100mg) which crystallised on standing, Mp 101-104°C.

EXAMPLE 3

This Example illustrates the preparation of 2-benzyl-5-methoxycarbonyl-3-isothiazolinone (Compound No 58 of Table I).

A solution of mercaptosuccinic acid (XXI, Z=H) (15g), cyclohexanone (15.5ml) and p-toluenesulphonic acid (100ml) in dry toluene (150ml) was heated to reflux in a Dean-Starke apparatus. After separation of water was complete (4 hours) the solvent was evaporated to give a yellow solid. Recrystallisation from diethyl ether-hexane afforded compound (XX, Z=H; $R^1$, $R^2$=$-(CH_2)_5-$) (19g, 83%) as a white crystalline solid, Mp 113-116°; $_{max}$ 3400-2400, 1760, 1710$cm^{-1}$.

Compound (XX, Z=H; $R^1$, $R^2$=$-(CH_2)_5-$) (5g) was dissolved in dry dichloromethane (50ml) and thionyl chloride (1.9ml) added dropwise. After stirring at room temperature for 3 hours, the solution was added dropwise to a solution of benzylamine (6ml) in dichloromethane (50ml) at room temperaure. After a further 3 hours, the reaction mixture was diluted with dichloromethane and washed successively with dilute hydrochloric acid, aqueous sodium bicarbonate and brine. The organic phase was dried over anhydrous magnesium sulphate, filtered and evaporated to give amide (XVIII, Z=H; $R^1$, $R^2$=$-(CH_2)_5-$; Y=$-CH_2Ph$) (4.41g, 70%) as an

oil which crystallised on standing, Mp109-110° ($CHCl_3$-$Et_2O$); $_{max}$ 3300, 1765, 1650cm$^{-1}$.

A solution of amide (XVIII, Z=H; $R^1$, $R^2$=-$(CH_2)_5$-; Y=-$CH_2Ph$) (10g) in 5% methanolic hydrogen chloride (200ml) was stirred at room temperature for 5 hours. TLC analysis showed quantitative conversion to a single more polar product. The reaction mixure was diluted with water and extracted with dichloromethane. The organic phases were combined, washed with aqueous sodium bicarbonate and dried over anhydrous magnesium sulphate. Evaporation afforded an amber oil (9.96g) which solidified on standing. The oil was redissolved in chloroform (100ml) and sulphuryl chloride (6.5ml) added dropwise at 0°C with stirring. After 6 hours the solvent was removed to give a yellow oil (13.01g). Crystallisation from diethyl ether-hexane afforded 2-benzyl-5-methoxycarbonyl-3-isothiazolinone, Mp 102-105°C.

## EXAMPLE 4

An emulsifiable concentrate was made up by mixing the ingredients, and stirring the mixture until all the constituents were dissolved.

| | |
|---|---|
| Compound No. 7 of Table I | 10 % |
| Ethylene dichloride | 40% |
| Calcium dodecylbenzenesulphate | 5% |
| "Lubrol" L | 10% |
| "Aromasol" H | 35% |

## EXAMPLE 5

A composition in the form of grains readily dispersible in a liquid, e.g. water, was prepared by grinding together the first three ingredients in the presence of added water and then mixing in the sodium acetate. The resultant mixture was dried and passed through a British

Standard mesh sieve, size 44-100, to obtain the desired size of grains.

| | |
|---|---|
| Compound No. 7 of Table I | 50% |
| "Dispersol" T | 25% |
| "Lubrol" APN5 | 1.5% |
| Sodium acetate | 23.5% |

EXAMPLE 6

The ingredients were all ground together to produce a powder formulation readily dispersible in liquids.

| | |
|---|---|
| Compound No. 7 of Table I | 45% |
| "Dispersol" T | 5% |
| "Lissapol" NX | 0.5% |
| "Cellofas" B600 | 2% |
| Sodium acetate | 47.5% |

EXAMPLE 7

The active ingredient was dissolved in a solvent and the resultant liquid was sprayed on to the granules of China clay. The solvent was then allowed to evaporate to produce a granular composition.

| | |
|---|---|
| Compound No. 7 of Table I | 5% |
| China clay granules | 95% |

EXAMPLE 8

A composition suitable for use as a seed dressing was prepared by mixing the three ingredients.

| | |
|---|---|
| Compound No. 7 of Table I | 50% |
| Mineral oil | 2% |
| China clay | 48% |

EXAMPLE 9

A dusting powder was prepared by mixing the active ingredient with talc.

| | |
|---|---|
| Compound No. 7 of Table I | 5% |
| Talc | 95% |

EXAMPLE 10

A Col formulation was prepared by ball-milling the constituents set out below and then forming an aqueous suspension of the ground mixture with water.

| | |
|---|---|
| Compound No. 7 of Table I | 40% |
| "Dispersol" T | 10% |
| "Lubrol" APN5 | 1% |
| Water | |

EXAMPLE 11

A dispersible powder formulation was made by mixing together the ingredients set out below and then grinding the mixture until all were thoroughly mixed.

| | |
|---|---|
| Compound No. 7 of Table I | 25% |
| "Aerosol" OT/B | 2% |
| "Dispersol" A.C. | 5% |
| China clay | 28% |
| Silica | 40% |

EXAMPLE 12

This Example illustrates the preparation of a dispersible powder formulation. The ingredients were mixed and the mixture then ground in a comminution mill.

| | |
|---|---|
| Compound No. 7 of Table I | 25% |
| "Perminal" BX | 1% |
| "Dispersol" T | 5% |
| Polyvinylpyrrolidone | 10% |
| Silica | 25% |
| China clay | 34% |

EXAMPLE 13

The ingredients set out below were formulated into a dispersible powder by mixing then griding the ingredients.

| | |
|---|---|
| Compound No. 7 of Table I | 25% |
| "Aerosol" OT/B | 2% |
| "Dispersol" A | 5% |
| China clay | 68% |

In Examples 4 to 13 the proportions of the ingredients given are by weight.

The remaining compounds set out in Table I above were formulated similarly to Examples 4 to 13.

There now follows an explanation of the compositions or substances represented by the various Trade Marks and Trade Names mentioned above.

| | |
|---|---|
| LUBROL L : | a condensate of nonyl phenol (1 mole) with ethylene oxide (13 moles) |
| AROMASOL H : | a solvent mixture of alkylbenzenes |
| DISPERSOL T & AC : | a mixture of sodium sulphate and a condensate of formaldehyde with sodium naphthalene sulphonate |

| | |
|---|---|
| LUBROL APN5 : | a condensate of nonyl phenol (1 mole) with naphthalene oxide (5.5 moles) |
| CELLOFAS B600 : | a sodium carboxymethyl cellulose thickener |
| LISSAPOL NX : | a condensate of nonyl phenol (1 mole) with ethylene oxide (8 moles) |
| AEROSOL OT/B : | dioctyl sodium sulphosuccinate |
| PERMINAL BX : | a sodium alkyl naphthalene sulphonate |

EXAMPLE 14

The compounds were tested againt a variety of mainly foliar fungal diseases of plants. The techniques employed were as follows.

The plants were grown in John Innes Potting Compost (No 1 or 2) in 4cm diameter minipots. The test compounds were formulated either by bead milling with aqueous Dispersol T or as a solution in acetone or acetone/ethanol which was diluted to the required concentration immediately before use. For the foliar diseases, solutions and suspensions (100ppm ai) were sprayed on the foliage and applied to the roots of the plant via the soil. The sprays were applied to maximum retention and the root drenches to a final concentration equivalent to approximately 40ppm ai/dry soil. Tween 20, to give a final concentration of 0.05%, was added when the sprays were applied to cereals (ai means "active ingredient").

Most were protectant tests where the compound was applied to the soil and roots and to the foliage one or two days before the plant was inoculated with the pathogen.

However, in the case of the test against <u>Erisyphe graminis hordei</u>, the treatment was eradicative and the compounds were applied one day after inoculation.

The foliar pathogens were applied as spore suspensions onto the leaves of the test plants.

After inoculation, the plants were placed in an appropriate environment to allow infection to proceed and then incubated until the disease was ready for assessment. The period between inoculation and assessment varied from four to fourteen days according to the disease and the environment.

Disease control was recorded using the following grading system.

4 = no disease
3 = trace to 5% of disease on untreated plants
2 = 6-25% of disease on untreated plants
1 = 26-59% of disease on untreated plants
0 = 60-100% of disease on untreated plants

An *in vitro* test was used to evaluate the compounds against *Botrytis cinerea*. Molten potato dextrose agar (7.5ml) was added to 2.5ml of chemical in 9cm plastic petri dishes, giving a final concentration of 25ppm ai per plate. After 24 hours a spore suspension of *Botrytis cinerea* was applied as single drops onto the agar. The plates were incubated at 25°C and inhibition of fungal growth was recorded using the following grading system after two days.

0 = no control
2 = partial control
4 = complete control

The results are shown in Table II.

TABLE II

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 1 | 3 | 2 | 0 | 4 | - | 0 | 3 |
| 2 | 3 | 0 | 0 | 4 | - | 0 | 2 |
| 3 | 0 | 1 | 0 | 4 | - | 0 | 4 |
| 4 | 2 | 0 | 0 | 3 | 0 | 2 | 1 |
| 5 | 0 | 0 | 0 | 4 | - | - | 0 |
| 6 | 0 | 0 | 0 | 4 | - | - | 0 |
| 7 | 0 | 0 | 0 | 4 | - | - | 0 |
| 8 | 0 | 0 | 0 | 4 | - | - | 0 |

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 9 | 0 | 0 | 0 | 4 | - | 0 | 0 |
| 10 | 0 | 0 | 0 | 3 | - | 0 | 0 |
| 11 | 2 | 0 | 0 | 3 | - | 0 | 3 |
| 12 | 0 | 0 | 0 | 4 | - | 0 | 1 |
| 13 | 0 | 0 | 0 | 4 | - | - | 3 |
| 14 | 1 | 0 | 0 | 3 | - | - | 1 |
| 15 | 0 | 0 | 0 | 4 | - | 0 | 3 |
| 16 | 1 | 0 | 0 | 4 | - | 0 | 3 |

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 17 | 0 | 2 | 0 | 4 | - | 0 | 0 |
| 18 | 0 | 0 | 0 | 4 | 0 | 0 | 3 |
| 19 | 0 | 0 | 0 | 4 | - | 0 | 1 |
| 20 | 0 | 0 | 0 | 4 | - | 0 | 0 |
| 21 | 0 | 0 | 3 | 4 | - | 0 | 1 |
| 22 | 4 | 0 | 0 | 3 | 0 | 1 | 0 |
| 23 | 0 | 0 | 0 | 4 | 0 | 3 | 2 |
| 24 | 0* | 0* | 0* | 3* | - | 4* | 0* |

* Result at 10ppm, foliar spray only

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 25 | 0* | 0* | 0* | 0* | – | 0* | 4* |
| 26 | 0 | 0 | 0 | 2 | 0 | 0 | 0 |
| 27 | 0 | 0 | 0 | 2 | – | 0 | 0 |
| 28 | 0 | 0 | 0 | 3 | – | 0 | 3 |
| 29 | 0 | 0 | 0 | 0 | – | 0 | 2 |
| 30 | 0 | 0 | 0 | 0 | – | 0 | 0 |
| 31 | 3 | 0 | 0 | 4 | – | 0 | 0 |
| 32 | 0 | 0 | 0 | 3 | – | 0 | 1 |

* Result at 10ppm, foliar spray only

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 33 | 0 | 0 | 0 | 4 | 2 | 0 | 0 |
| 34 | 0 | 0 | 0 | 0 | - | 0 | 0 |
| 35 | 0 | 0 | 0 | 4 | 2 | 0 | 0 |
| 36 | 0 | 0 | 0 | 2 | - | 1 | 0 |
| 37 | 0 | 0 | 0 | 3 | - | 1 | 2 |
| 38 | 0 | 0 | 0 | 3 | - | 0 | 0 |
| 39 | 2 | 0 | 0 | 4 | - | 0 | 0 |
| 40 | 0 | 0 | 0 | 4 | 0 | 0 | 0 |

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 41 | 4 | 0 | 0 | 2 | 0 | 2 | 0 |
| 42 | 0 | 0 | 0 | 4 | - | 0 | 0 |
| 43 | 0 | 2 | 0 | 4 | 4 | - | 3 |
| 44 | 0 | 0 | 0 | 4 | - | 2 | 1 |
| 45 | 0 | 0 | 0 | 4 | - | 0 | 3 |
| 46 | 0* | 0* | 0* | 3* | 0 | 2* | 1 |
| 47 | 0 | 0 | 0 | 4 | 4 | 0 | 0 |
| 48 | 0 | 0 | 0 | 4 | - | 0 | 1 |

* Result at 10ppm, foliar spray only

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 49 | 0 | 0 | 0 | 4 | 0 | 0 | 2 |
| 50 | 0 | 0 | 0 | 4 | 0 | M | 0 |
| 51 | 0 | 0 | 0 | 3 | 0 | 0 | 0 |
| 52 | 0 | 0 | 0 | 3 | 2 | 0 | 0 |
| 53 | 0* | 0* | 0* | 4* | - | 0* | 0* |
| 54 | 0 | 0 | 0 | 3 | 0 | 0 | 0 |
| 55 | 0 | 0 | 0 | 3 | 0 | 0 | 4 |
| 56 | 0 | 0 | 0 | 3 | 2 | 0 | 2 |

* Result at 25ppm, foliar spray only

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 25PPM) | CERCOSPORA ARACHIDICOLA (PEANUT) | VENTURIA INEAQUALIS (APPLE) |
|---|---|---|---|---|---|---|---|
| 57 | 0 | 0 | 0 | 3 | 0 | 0 | 4 |
| 58 | 0 | 0 | 0 | 0 | 2 | 0 | 0 |
| 59 | 0 | 0 | 0 | 0 | 0 | 0 | 0 |
| 60 | 0 | 0 | 0 | 3 | 0 | 0 | 3 |
| 61 | 0 | 0 | 0 | 0 | 0 | 0 | 3 |
| 62 | 0 | 0 | 0 | 4 | 2 | 0 | 0 |
| 63 | 0 | 0 | 0 | 2 | 0 | 0 | 0 |
| 64 | 0 | 0 | 0 | 4 | 0 | 0 | 0 |

TABLE II (CONT/D)

| COMPOUND NO | PUCCINIA RECONDITA (WHEAT) | ERYSIPHE GRAMINIS (BARLEY) | PIRICULARIA ORYZAE (RICE) | PLASMOPARA VITICOLA (VINE) | BOTRYTIS CINEREA (IN VITRO 6.25PPM) | CERCOSPORA ARACHIDICOLA (PEANUT) | VENTURIA INEAQUALIS (APPLE) |
|---|---|---|---|---|---|---|---|
| 65 | 0* | 0* | 0* | 0* | 0 | 0* | 0* |
| 66 | 0* | 0* | 0* | 4* | 0 | 0* | 0* |
| 67 | 0 | 0 | 0 | 4 | - | 0 | 2 |
| 68 | 0 | 0 | 0 | 4 | - | 0 | 1 |
| 69 | 0 | 0 | 0 | 3 | - | 0 | 0 |
| 70 | 0 | 0 | 0 | 4 | - | 0 | 0 |

* Results at 25ppm, foliar spray only

PP 33842
HGHA/dlc
10 Mar 87

# Claims

1. A compound having the general formula (I):

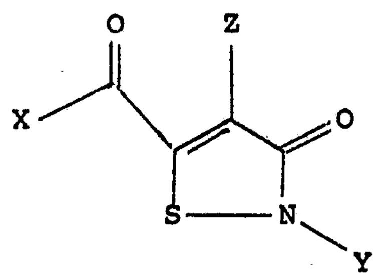

wherein X is an optionally substituted aryl (especially phenyl), aralkyl, alkyl, hydroxy, alkoxy, aryloxy, amino, alkylamino, dialkylamino, or a heterocyclic group· or a hydrogen atom: Y is an optionally substituted aralkyl (especially benzyl), aryl, alkenyl, alkyl or substituted alkyl group, or a hydrogen atom; and Z is an alkyl or cyano group, or a hydrogen or halogen atom; provided that when X is phenyl and Z is hydrogen Y is not methyl, phenyl, 4-methylphenyl, 4-hydroxyphenyl, 4methoxyphenyl, 4-chlorophenyl, 3- or 4-nitrophenyl, or benzyl.

2. A compound as claimed in claim 1 wherein X and Y, when they represent aryl or aralkyl groups, are phenyl or benzyl optionally substituted with halogen, alkyl, haloalkyl, alkoxy, cyano or nitro.

3. A compound as claimed in claim 2 wherein X and Y which may be the same or different, are phenyl; 2-, 3- or 4-chlorophenyl: 2,3-, 2,6-, 3,4- or 3,5-dichlorophenyl; 2-,3- or 4-fluorophenyl; 2,4-, 2,6-, 3,4- or 3,5-difluorophenyl; 2-,3- or 4- bromophenyl; 2-,3- or 4-methoxyphenyl; 2,4-, 2,6-, 3,4-, or 3,5-

dimethoxyphenyl; 2-,3- or 4-nitrophenyl; 2-,3- or 4-methylphenyl; 2-,3- or 4-trifluoromethylphenyl; benzyl; 2-,3- or 4- chlorobenzyl; 2,4-, 2,6-, 3,4- or 3,5-dichlorobenzyl; 2-, 3- or 4-fluorobenzyl; 2,4-, 2,6-, 3,4-, 3,5- difluorobenzyl; 2-, 3-, or 4-bromobenzyl; 2-, 3- or 4- methoxybenzyl; 2,4-, 2,6-, 3,4-, or 3,5- dimethoxybenzyl; 2-, 3- or 4-nitrobenzyl; 2-, 3- or 4- methylbenzyl; and 2-, 3- or 4-trifluoromethylbenzyl; 2- or 3- pyridylmethyl; 2- or 3-furanylmethyl; or 2- or 3- thiophenylmethyl.

4. A compound as claimed in claim 1 wherein when X, Y or Z is alkyl or contains an alkyl moiety as in alkoxy alkylamino or dialkylamino, the alkyl moiety is optionally substituted and is a cyclic, straight or branched chain alkyl group having 1 to 10 carbon atoms or any combination thereof.

5. A compound as claimed in claim 4 wherein the alkyl moiety is methyl, ethyl, propyl (N- or iso-propyl) or butyl (n-, sec-, iso- or t-butyl).

6. A compound as claimed in claim 4 or claim 5 wherein the alkyl moiety is substituted with halogen, aryl, alkoxy or hydroxy.

7. A process for preparing the compounds of the invention having the general formula (I) :

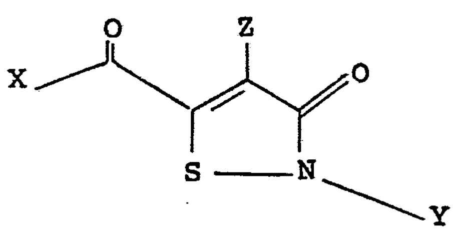

(I)

wherein X is an optionally substituted aryl (especially phenyl), aralkyl, alkyl or heterocyclic group as defined in claim 1 and Y and Z are as defined in claim 1 which comprises the treatment of gamma-keto-amides of general formula (II):

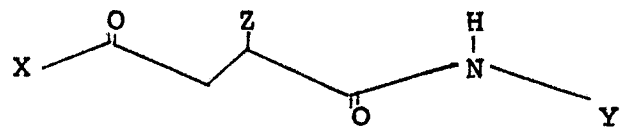

(II)

wherein X is an optionally substituted aryl (especially phenyl), aralkyl, alkyl or heterocyclic group as defined above, and Y and Z are as defined above, with thionyl chloride in the presence or absence of an acid binding agent (such as potassium carbonate or sodium bicarbonate) with or without a convenient co-solvent (such as chloroform or dichloromethane) and at a convenient temperature (such as 0 to 80°C).

8. A process for preparing the compounds of the invention having the general formula (I), wherein X is an alkoxy, aryloxy, alkylamino or dialkylamino group, and Y and Z are as defined in claim 1 which comprises esterifying a carboxylic acid of the general formula (XV):

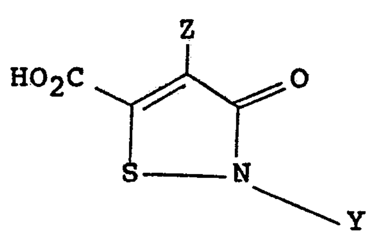

(XV)

wherein Y and Z are as defined in claim 1.

9. A process for preparing the compounds of the invention having the general formula (I), wherein X and Y are as defined in claim 1 and Z is a halogen atom, which comprises treating a compound of general formula (XXVI):

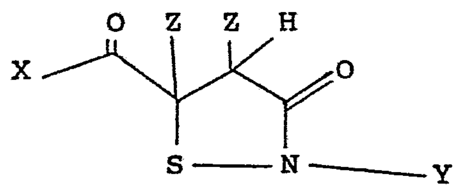

(XXVI)

wherein X and y are as defined in claim 1 and Z is a halogen atom, with an acid-binding agent (such as triethylamine) in a convenient solvent (such as chloroform or dichloromethane).

10. A fungicidal or plant growth regulating composition comprising, as an active ingredient, a compound of general formula (I) as defined in any of claim 1 to 6 and a carrier or diluent therefor.

11. A method of combating fungi or regulating plant growth which comprises applying to a plant, to seed of a plant, or to the locus of the plant or seed, a compound as defined in any of claims 1 to 6.

0246735

European Patent Office

# EUROPEAN SEARCH REPORT

Application number

EP 87 30 2346

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | CHEMICAL ABSTRACTS, vol. 99, no. 11, 12th September 1983, page 562, abstract no. 88100k, Columbus, Ohio, US; Yu.L. ZBOROVSKII et al.: "Heterocyclization of acetylenedicarboxylic acid in the presence of sulfur dioxide and hydrogen bromide", & ZH. ORG. KHIM. 1983, 19(6), 1337-8 * Abstract * | 1 | C 07 D 275/02<br>C 07 D 417/06<br>A 01 N 43/80<br>C 07 F 9/65 |
| X | J. HETEROCYCL. CHEM., vol. 21, no. 6, November/December 1984, pages 1679-1683; A. TSOLOMITIS et al.: "Ring transformation of 5-aroyl-3(2H)-isothiazolones to 1,2,5-oxathiazole and 1,2,3-thiadiazole derivatives [1]" * Pages 1679,1682 * | 1,7 | TECHNICAL FIELDS SEARCHED (Int. Cl 4) |
| X | J. HETEROCYCL. CHEM., vol. 22, no. 6, November/December 1985, pages 1635-1637; A. TSOLOMITIS et al.: "The rearrangement of 2-benzyl-5-mesitoyl-3(2H)-isothiazolone to 2-phenyl-6-mesitoyl-3,4-dihydro-1,3-thiazin-4(2H)-one" * Page 1635, scheme 2; page 1637 * | 1,2,7 | C 07 D 275/00<br>C 07 D 417/00<br>A 01 N 43/00<br>C 07 F 9/00 |

--- -/-

The present search report has been drawn up for all claims

| Place of search | Date of completion of the search | Examiner |
|---|---|---|
| THE HAGUE | 05-08-1987 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

0246735

European Patent Office

# EUROPEAN SEARCH REPORT

Application number

EP 87 30 2346



## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,X | J. HETEROCYCL. CHEM., vol. 17, no. 7, November 1980, pages 1645-1646; A. TSOLOMITIS et al.: "Formation of 5-aroyl-3-(2H)isothiazolones from the reaction of 3-aroylpropionamides with thionyl chloride (1)" * Whole article * --- | 1-3,7 | |
| D,X | TETRAHEDRON, vol. 37, no. 22, 1981, pages 3867-3870, Pergamon Press Ltd, Oxford, GB; R.J.S. BEER et al.: "Studies on 5-benzoyl-3-isothiazolinones" * Pages 3867,3869 * ----- | 1,7 | |

TECHNICAL FIELDS SEARCHED (Int. Cl 4)

The present search report has been drawn up for all claims

| Place of search | Date of completion of the search | Examiner |
|---|---|---|
| THE HAGUE | 05-08-1987 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82